(19) Europäisches Patentamt
European Patent Office
Office européen des brevets

(11) Veröffentlichungsnummer : **0 432 435 B1**

# (12) EUROPÄISCHE PATENTSCHRIFT

(45) Veröffentlichungstag der Patentschrift :
**02.02.94 Patentblatt 94/05**

(51) Int. Cl.⁵ : **C07C 69/67,** C07C 45/50, C07C 47/02

(21) Anmeldenummer : **90120998.1**

(22) Anmeldetag : **02.11.90**

## (54) Verfahren zur Herstellung von 2-Formyl-2-methyl-bernsteinsäureestern.

(30) Priorität : **16.11.89 DE 3938092**

(43) Veröffentlichungstag der Anmeldung :
**19.06.91 Patentblatt 91/25**

(45) Bekanntmachung des Hinweises auf die Patenterteilung :
**02.02.94 Patentblatt 94/05**

(84) Benannte Vertragsstaaten :
**BE DE FR GB IT NL**

(56) Entgegenhaltungen :
**DE-C- 1 793 069**

(56) Entgegenhaltungen :
**CHIMIA, Band 40, 1986, Basel, L. KOLLAR et al: "Hydroformylation of Unsaturated Dicarboxylic Esters with Rhodium Containing Catalysts", Seite 428/9**

(73) Patentinhaber : **BASF Aktiengesellschaft**
**Carl-Bosch-Strasse 38**
**D-67063 Ludwigshafen (DE)**

(72) Erfinder : **Bertleff, Werner, Dr.**
**Franz-Marc-Strasse 12**
**W-6806 Viernheim (DE)**
Erfinder : **Butz, Gerhard**
**Sandstrasse 4**
**W-6745 Offenbach (DE)**

**EP 0 432 435 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von 2-Formyl-2-methylbernsteinsäureestern durch Umsetzung von Itaconsäureestern mit Kohlenmonoxid und Wasserstoff mittels eines Katalysatorsystems aus Rhodium und einer tertiären Phosphorverbindung bei erhöhtem Druck und erhöhter Temperatur.

Die Hydroformylierung olefinisch ungesättigter Verbindungen nach dem sogenannten Niederdruckverfahren mittels eines Katalysatorsystems aus Rhodium und im Überschuß vorliegenden tertiären Phosphorverbindungen ist allgemein bekannt, z.B. aus der DE-OS 17 93 069. Als Reaktionsbedingungen werden hierbei eine Temperatur von etwa 50 bis 145°C und ein Druck von bis zu etwa 31,5 bar empfohlen.

Die Herstellung von 2-Formyl-2-methyl-bernsteinsäureestern (Ia) und daneben der isomeren 2-Formylmethyl-bernsteinsäureester (Ib) aus dem Itaconsäureester (II) nach diesem Verfahren

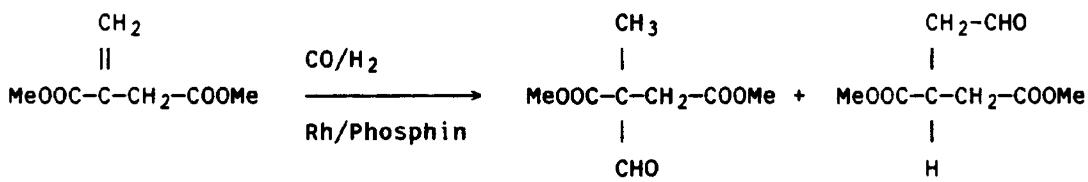


II Ia Ib

ist Gegenstand der Arbeit von Kollár et al (Chimia 40 (1986) 428).

Man erhält hiernach den Ester Ia mit Rhodium/Triphenylphosphin-Katalysatorsystemen bei 100°C und Partialdrücken für CO und $H_2$ von jeweils 40 bar (Gesamtdruck 80 bar) in Ausbeuten von etwa 37 bis 42 %. Bei Verwendung des optisch aktiven (4R,5R)-(-)-4,5-Bis-(diphenylphosphinomethyl)-2,2-dimethyll,3-dioxolan ("(R,R)-DIOP") erzielt man unter den gleichen Bedingungen eine Ausbeute von 73 %, wobei eines der optischen Isomeren von Ia im Überschuß gebildet wird.

Diese unbefriedigenden Ausbeuten sind darauf zurückzuführen, daß bis zu 45 % des eingesetzten Itaconsäuremethylesters in einer Nebenreaktion zum Methylbernsteinsäuremethylester hydriert werden. Lediglich im Falle des teuren "DIOP" beträgt der Anteil an dem Methylbernsteinsäuremethylester nur 10 %, jedoch ist auch in diesem Falle die Ausbeute an Ia unbefriedigend.

Der Erfindung lag daher die Aufgabe zugrunde, die 2-Formyl-2-methylbernsteinsäureester durch Hydroformylierung von Itaconsäureestern besser zugänglich zu machen als bisher.

Demgemäß wurde ein verbessertes Verfahren zur Herstellung von 2-Formyl-2-methyl-bernsteinsäureestern durch Umsetzung von Itaconsäureestern mit Kohlenmonoxid und Wasserstoff mittels eines Katalysatorsystems aus Rhodium und einer tertiären Phosphorverbindung bei erhöhtem Druck und erhöhter Temperatur gefunden, welches dadurch gekennzeichnet ist, daß man es bei einem Druck von 90 bis 325 bar vornimmt.

Das gute Gelingen dieses Verfahrens ist von der Art der eingesetzten Itaconsäureester nach den bisherigen Beobachtungen praktisch nicht abhängig, so daß die Esterreste prinzipiell beliebig sein können. Da die Verfahrensprodukte größenteils als Bausteine für die Herstellung von Polykondensations- und Polyadditionsprodukten dienen, wobei die Esterreste ohnehin wieder abgespalten werden, werden die Methyl-, die Ethyl-, die Benzyl- und die Phenylgruppe als Esterreste besonders bevorzugt. Daneben sind zu nennen

- $C_1$-$C_{10}$-, vor allem $C_1$-$C_4$-Alkylgruppen allgemein
- Arylgruppen allgemein
- Aralkylgruppen allgemein und
- Cycloalkylgruppen wie die Cyclopentyl- und Cyclohexylgruppe,

wobei diese Gruppen ihrerseits Substituenten tragen können, die sich unter den Reaktionsbedingungen inert verhalten, wie beispielsweise Fluor, Chlor und Brom, $C_1$-$C_4$-Alkylgruppen, $C_1$-$C_4$-Alkoxygruppen, die Hydroxylgruppe, die Cyangruppe und die Nitrogruppe.

Erfindungsgemäß nimmt man die Umsetzung bei einem Druck von 90 bis 325 bar vor, wobei sich ein Druck von 100 bis 280, besonders 100 bis 160 bar, empfiehlt. Dieser Gesamtdruck entspricht praktisch der Summe der Partialdrücke des Kohlenmonoxids und des Wasserstoffs. Das Partialdruckverhältnis und damit das Molverhältnis von CO zu $H_2$ beträgt vorzugsweise 0,1 : 1 bis 10 : 1, vor allem 0,8 : 1 bis 1,2 : 1.

Im übrigen gleicht das Verfahren der üblichen Technik für die kontinuierliche oder diskontinuierliche Hydroformylierung olefinisch ungesättigter Verbindungen mittels Rhodium und tertiären Phosphorverbindungen

als Katalysatorsystem, so daß detaillierte Ausführungen hierüber entbehrlich sind. Das Rhodium kann in Form von Komplexen, z.B. als $HRh(CO)(PPh_3)_3$, oder in Form eines beliebigen, insbesondere halogenfreien Salzes eingesetzt werden. Vorzugsweise verwendet man das Rhodium (als Metall gerechnet) in Mengen von 100 bis 400 mg pro kg des Itaconsäureesters.

Als tertiäre Phosphorverbindungen kommen in erster Linie Triarylphosphine wie vor allem Triphenylphosphin und Alkyldiarylphosphine wie Hexyldiphenylphosphin, daneben aber auch Trialkylphosphine wie Tributylphosphin sowie Triarylphosphite wie Triphenylphosphit und Trialkylphosphite wie Triethylphosphit in Betracht.

Das Molverhältnis dieser Liganden zum Rhodium liegt vorzugsweise im Bereich von 20 : 1 bis 500 : 1, besonders bevorzugt im Bereich 40 : 1 bis 250 : 1.

Es ist zweckmäßig, die Hydroformylierung bei 50 bis 150°C vorzunehmen, und zwar besonders bei 70 bis 90°C, weil bei diesen Temperaturen nur sehr wenige Nebenprodukte anfallen.

Besonders gut gelingt die Hydroformylierung in Abwesenheit eines Lösungsmittels, weil sich in diesem Falle die Hydrierung der Itaconsäureester weniger störend bemerkbar macht. Zieht man es aus verfahrenstechnischen Gründen vor, in Gegenwart eines Lösungsmittels zu arbeiten, so eignen sich hierzu unter den Reaktionsbedingungen inerte Lösungsmittel wie Toluol, Xylol, Dioxan oder Carbonsäureester wie Ethylacetat oder Methylbernsteinsäureester in Mengen von etwa 0,5 bis 2 kg/pro kg des Itaconsäureesters. Unter den angegebenen Reaktionsbedingungen betragen die Reaktionszeiten für einen praktisch quantitativen Umsatz etwa 2 bis 4 Stunden. Man erhält das gewünschte Verfahrensprodukt dabei in der Regel in Ausbeuten zwischen 70 und 85 %. Daneben entstehen noch etwa 1 bis 10 % der isomeren Verbindung vom Typ Ib. Der Rest entfällt größtenteils auf die Methylbernsteinsäureester. Alle Verbindungen lassen sich wie üblich destillatlv voneinander trennen.

Die Verfahrensprodukte sind wichtige Zwischenprodukte für die Herstellung von Kunststoffen. Die Hydrierung der Aldehydfunktion liefert 2-Hydroxymethyl-2-methyl-bzw. 2-Hydroxyethyl-bernsteinsäureester, die wichtige Bausteine für Polyester sind. Bei der Perhydrierung erhält man die entsprechenden Triole, welche als Vernetzungskomponenten in der Polyester- und Polyurethanchemie dienen.

Beispiel

In einem 2,5-1-Hubrührautoklaven wurde eine Mischung aus 700 g Dimethylitaconat, 1,4 g $HRh(CO)(PPh_3)_3$ und 15,7 g $PPh_3$ bei 85°C und unter einem Druck von 100 bar eines äquimolaren $CO/H_2$-Gemisches für 3 h der Hydroformylierungsreaktion unterworfen. Nach gaschromatographischer Analyse des Reaktionsgemisches fielen die Verfahrensprodukte in folgenden Ausbeuten an:

| | |
|---|---|
| 2-Formyl-2-methyl-dimethylsuccinat: | 81 % |
| 2-Formylmethyl-dimethylsuccinat: | 1,6 % |
| 2-Methyl-dimethylsuccinat: | 14,6 % |

## Patentansprüche

1. Verfahren zur Herstellung von 2-Formyl-2-methyl-bernsteinsäureestern durch Umsetzung von Itaconsäureestern mit Kohlenmonoxid und Wasserstoff mittels eines Katalysatorsystems aus Rhodium und einer tertiären Phosphorverbindung bei erhöhtem Druck und erhöhter Temperatur, dadurch gekennzeichnet, daß man es bei einem Druck von 90 bis 325 bar vornimmt.

2. Verfahren nach Anspruch 1, dadurch gekennzeichnet, daß man die Reaktion bei 100 bis 280 bar durchführt.

3. Verfahren nach den Ansprüchen 1 oder 2, dadurch gekennzeichnet, daß man die Reaktion bei 70 bis 90°C durchführt.

## Claims

1. A process for the preparation of 2-formyl-2-methylsuccinic esters by reacting itaconic esters with carbon

monoxide and hydrogen by means of a catalyst system comprising rhodium and a tertiary phosphorus compound at elevated temperature and at a superatmospheric pressure of from 90 to 325 bar.

2. A process as claimed in claim 1, wherein the reaction is carried out at from 100 to 280 bar.

3. A process as claimed in claim 1 or 2, wherein the reaction is carried out from 70 to 90°C.

## Revendications

1. Procédé de préparation d'esters de l'acide 2-formyl-2-méthylsuccinique par réaction d'esters de l'acide itaconique avec le monoxyde de carbone et l'hydrogène au moyen d'un système catalytique au rhodium et d'un composé du phosphore tertiaire sous une pression élevée et à une température élevée, caractérisé en ce que l'on procède à une pression de 90 à 325 bar.

2. Procédé selon la revendication 1, caractérisé en ce que l'on effectue la réaction à une pression comprise entre 100 et 280 bar.

3. Procédé selon la revendication 1 ou 2, caractérisé en ce que l'on effectue la réaction à une température de 70 à 90°C.